# EUROPEAN PATENT SPECIFICATION

(11) **EP 1 148 048 B1**
(45) Date of publication and mention of the grant of the patent: **29.06.2005**
(21) Application number: 01117751.6
(22) Date of filing: 01.04.1998
(51) Int. Cl.: C07C 275/40, C07C 335/20, C07C 311/29, C07C 311/20, A61K 31/17, A61K 31/63

(54) **Polyaromatic compounds for treating herpes viral infections**
Polyaromatische Verbindungen zur Behandlung von Herpes-Infektionen
Composés polyarômatiques por le traitement d'une infection herpes.

(30) Priority: 10.04.1997 US 43232 P
(43) Date of publication of application: 24.10.2001
(62) Divisional of application: 98913241.0
(73) Proprietor: Pharmacia & Upjohn Company LLC, Kalamazoo, MI 49001 (US)
(72) Inventor: Barbachyn, Michael R., East Hampton, Connecticut 06424 (US); Homa, Fred L., Kalamazoo, Michigan 49008 (US); Monge, Antonio, Cizur Menor (Navarra) (ES); Santiago, Esteban, 31003 Pamplona (ES); Martinez-Irujo, Juan J., 31003 Pamplona (ES); Font, Maria, 31001 Pamplona (ES)
(74) Representative: Perry, Robert Edward

(56) References cited:
- EP-A- 0 467 185
- US-A- 4 024 183
- DATABASE CROSSFIRE BEILSTEIN [Online] BEILSTEIN INSTITUT ZUR FOEDERUNG DER CHEMISCHEN WISSENSCHAFTEN, FRANKFURT AM MAIN, DE; Database-Accession no. 3519368 (BRN), XP002180334 & JOURNAL OF THE CHEMICAL SOCIETY., vol. 87, 1905, page 1308 LETCHWORTH GB
- DATABASE CROSSFIRE BEILSTEIN [Online] BEILSTEIN INSTITUT ZUR FOEDERUNG DER CHEMISCHEN WISSENSCHAFTEN, FRANKFURT AM MAIN, DE; Database-Accession no. 2913004, XP002180335 & MAKROMOL. CHEM., vol. 175, 1974, pages 3425-3432,
- I. B. HANNOUT ET AL.: "Synthesis of some new disulphonamides active against cotton leaf worm" JOURNAL FUER PRAKTISCHE CHEMIE, vol. 316, no. 5, 1974, pages 866-874, XP000926672 LEIPZIG, DE ISSN: 0021-8383
- CHEMICAL ABSTRACTS, vol. 119, no. 21, 22 November 1993 (1993-11-22) Columbus, Ohio, US; abstract no. 225647s, LIU, ZHAOPENG ET AL.: "Antiviral agents." page 980; column 1; XP002180333 & SHANDONG YIKE DAXUE XUEBAO, vol. 31, no. 1, 1993, pages 65-67,

## Description

### FIELD OF THE INVENTION

The present invention relates to polyaromatic compounds having useful antiviral activity against viruses of the herpes family, to a composition containing them, and to a method of using them for the treatment of herpes viral infections.

### BACKGROUND OF THE INVENTION

Viruses are made of nucleic acid (DNA or RNA) enclosed in a protein coat and sometimes further wrapped in a membranous envelope. Viruses are obligate intracellular parasites; they can only reproduce within a host cell. An isolated virus is unable to replicate itself, or do anything else for that matter, except infect an appropriate host cell. Of the DNA viruses, the herpes family is the source of the most common viral illnesses in man. The group consists of herpes simplex viruses type-1 and type-2 (HSV-1 and HSV-2), varicella zoster virus (VZV), cytomegalovirus (CMV), and Epstein-Barr virus (EBV). We have discovered that certain polyaromatic compounds characterized by formula II have potent antiviral activity against the herpes family, particularly against herpes simplex viruses. These compounds inhibit the origin-specific DNA-binding protein, an essential herpes virus replication protein, binding to the origin of viral DNA replication. As such, the compounds inhibit the initiation of herpes viral DNA synthesis in the host cell. Because of this unique mechanism, the compounds not only exhibit potent activity against herpes viruses but also are active against viral strains resistant to currently available therapeutic agents.

### INFORMATION DISCLOSURE

European Patent Application 0,611,754 A1 discloses derivatives of dimerized thiourea having the structure: 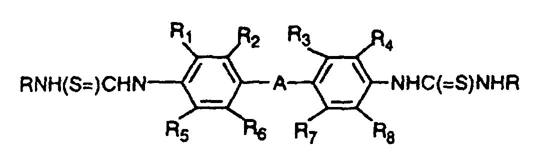 wherein
A is, among others, CH₂, S, O, or SO₂;
R is, among others, a substituted or unsubstituted aryl group having 6 to 30 carbon;
R₁, R₂, R₃, R₄, R₅, R₆, R₇, and R₈ are the same and different and are C₁₋₆ alkyl, NO₂, CN, H or halogen. The compounds are disclosed as being useful to prepare a near infrared resin material.

UK Patent Application GB 2,290,626 discloses, among others, bisurea compound of the structure: 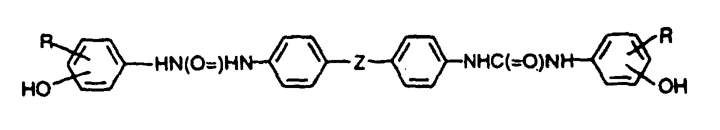 wherein
Z is CH₂, O, SO₂ or NH;
R is H, halogen, NO₂, C₁₋₁₂ alkyl, C₁₋₆ haloalkyl or C₁₋₆ alkoxy. Such compounds are described as being useful to prepare a thermal recording material.

U.S. Patent 5,500,322 discloses, among others, a developer additive having the structure: 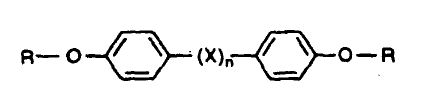 wherein
X is, among others, S, SO₂, O or CH₂;
R is an alkyl group, an alkenyl group or an arylalkyl group. Such compounds are described as being used in a toner composition.

European Patent Application 0,519,702 A1 discloses, among others, compound of the structure: 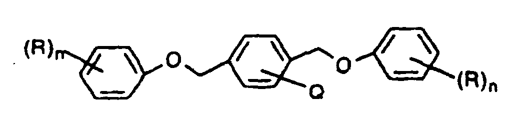 wherein
Q is H, halogen, nitro, C₁₋₆ alkyl, C₁₋₆ perhaloalkyl, C₁₋₆ alkylthio or C₁₋₆ alkyl sulfonyl;
R is, among others, C₁₋₁₀ alkyl, alkoxy, or halogen;
n is one to three. Such compounds are described as being active against picornaviruses including enteroviruses and rhinoviruses.

J. Chem. Soc. 87, (1905) 1308, XP-002180334; Makromol. Chem., 175 (1975), 3425-32, XP-002180335; J. Prakt. Chem. 316, 866-74 (1974); CA 119, 21 (1993), XP-002180333; and US-A-4024183 disclose compounds having a structure of or related to formula II (bellow).

### Summary of the Invention

According to the present invention, a compound for treating herpes viral infections is of formula II 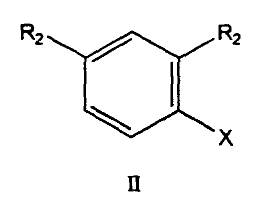 or a pharmaceutically acceptable salt thereof, wherein:
R₂ is
   (a) 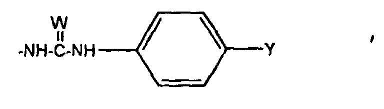 or
   (b) 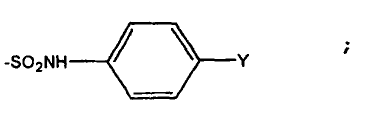
W is
   (a) =O, or
   (b) =S;
X is
   (a) -H,
   (b) -CH₃, or
   (c) -OCH₃; and
Y is
   (a) -CH₃,
   (b) -Cl, or
   (c) -NO₂.

Compounds of the invention are useful for the treatment of herpes viral infections.

Examples of compounds of the invention are
*N*,*N*"-(4-methoxy-1,3-phenylene)bis[*N*'-(4-nitrophenyl)urea],
*N*,*N*"-(4-methoxy-1,3-phenylene)bis[*N*'-(4-chlorophenyl)urea],
*N*,*N*"-(4-methoxy-1,3-phenylene)bis[*N*'-(*p*-tolyl)urea],
*N*,*N*"-(4-methyl-1,3-phenylene)bis[*N*'-(4-chlorophenyl)thiourea],
*N*,*N*"-(4-methoxy-1,3-phenylene)bis[*N*'-(4-chlorophenyl)thiourea],
*N*,*N*"-(4-methoxy-1,3-phenylene)bis[*N*'-(4-nitrophenyl)thiourea], and
*N*,*N*"-(1,3-phenylene)bis[*N*'-(*p*-tolyl)aminosulfonyl].

### DETAILED DESCRIPTION OF THE INVENTION

The present invention relates to polyaromatic compounds, their compositions and their use in medical therapy for the treatment of herpes viral infections including herpes simplex viruses type-1 and type-2 (HSV-1 and HSV-2), varicella zoster virus (VZV), cytomegalovirus (CMV), and Epstein-Barr virus (EBV). The compounds of the present invention are topically administered to the surface lesion caused by the herpes viruses.

Herpes viruses infections are recurrent infections characterized by the appearance on the skin or mucous membranes of multiple clusters of small vesicles, filled with clear fluid on slightly raised inflammatory bases. The present invention can also be applied prophylactically to prevent further recurrences of the viral lesions.

Patients of the present invention are chosen from those having been diagnosed with primary or recurrent herpes simplex type 1 or 2, herpes zoster, genital warts, chickenpox, or herpes keratitis. Such diseases and conditions are well known and readily diagnosed by physician of ordinary skill.

Topical administration means the direct contact of the active agents with the surface lesion such as, for example, by drops, sprays, ointments, lotions, creams or soaps.

The active compound or its composition is applied 1 to 5 times daily until the sore, lesion, and accompanying discomfort abates and essentially disappears.

The pharmaceutical compositions of this invention may be prepared by employing conventional technique to combine the compounds of formula II of this invention with a pharmaceutically acceptable carrier, and optionally, with pharmaceutically acceptable adjuvants and excipients employing standard and conventional techniques. The topical formulations may also desirably include a material which enhances absorption or penetration of the compounds of formula II through the skin or other affected areas.

The pharmaceutically acceptable carrier refers to any compatible non-toxic material suited for mixing with the active compounds of the present invention.

The quantity of active component, compounds of formula II, in a pharmaceutical composition may be varied or adjusted widely depending upon the requirements of the patient, the severity of viral infections, the potency of the particular compound being used, the particular formulation and the desired concentration. Generally, the quantity of active component will range between 0.05% to 25% by weight of the composition, preferably between 0.1% to 10% by weight of the composition.

For infections of the external tissues, e.g., mouth, eyes and skin, the formulations are preferably applied as a topical ointment or cream containing the active ingredient in an amount of, for example, 0.05 to 25% by weight of composition, preferably 0.1 to 10% w/w. When formulated in an ointment, the active ingredients may be employed with either a paraffinic or a water-miscible ointment base. Alternatively, the active ingredients may be formulated in a cream with an oil-in-water cream base.

Formulations suitable for topical administration in the eyes include eye drops wherein a compound of formula II is dissolved or suspended in a suitable carrier, especially an aqueous solvent. The active ingredient is preferably present in such formulations in a concentration of 0.05 to 25%, advantageously 0.5 to 10%.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

The term "pharmaceutically acceptable salts" refers to salts useful for administering the compounds of this invention and include hydrochloride, hydrobromide, hydroiodide, sulfate, phosphate, acetate, propionate, lactate, mesylate, maleate, malate, succinate, tartrate, citric acid, 2-hydroxyethyl sulfonate, fumarate and the like. These salts may be in hydrated form.

Compounds of formula II of the present invention are evaluated in an assay to measure the inhibition of HSV-1 viral replication in the presence of drug over a 14-16 hour period. The assay is discussed in detail by Pritchard, M.N. et al. "A Microliter Virus Yield Reduction Assay for the Evaluation of Antiviral Compounds Against Human Cytomegalovirus and Herpes Simplex Virus", *J*. *Virol*. *Meth.,* Vol. 28, pp 101-106 (1990). Briefly, 80% confluent monolayers of Vero cells in 24 well plates are infected with low multiplicities of HSV-1 (MOI = .001). After an absorption period of 1 hour at 37 °C, the monolayers are rinsed with culture media and aliquots of compounds diluted into tissue culture media are added for 14-16 hours at 37 °C in a CO₂ atmosphere. The growth media is removed and 1 ml of fresh media is added to each well and plates are placed at -70 °C. After a 1-5 day storage period the plates are rapidly thawed, samples are collected on ice and titrated on fresh Vero cell monolayers. Viral plaques are stained with 0.1% crystal violet in 20% alcohol for 10 minutes and are counted. Data are calculated to reflect the percentage of inhibition at indicated concentration relative to infected, non-drug treated controls. Results are shown in Table 1.

In the Table, the sign "++" refers to > 50% inhibition at the indicated concentration. The sign "+" refers to <50% inhibition at the indicated concentration. The sign "-" refers that no inhibitory activity is observed at the indicated concentration.

**TABLE 1**

| % Inhibition Against HSV-1 Viral Replication | | |
|---|---|---|
| Example No. | 30 µm | 5 µm |
| 1 | + | - |
| 2 | ++ | - |
| 3 | + | + |
| 4 | + | + |
| 5 | + | - |
| 6 | + | - |
| 7 | ++ | + |

Compounds of formula II may be prepared by the procedures shown in the schemes. As shown in SCHEME 1A, diamine 1 (when necessary it can be generated from the corresponding acid addition salt by treatment *in situ* with triethylamine) can be reacted with two equivalents of an appropriate isocyanate or isothiocyanate wherein R is R₁, R₂, R₃, or R₄ and is as defined above; X is as defined above. The reaction occurs in a suitable solvent system, such as anhydrous dioxane, at room temperature to generate the bisurea (Y = O) or bisthiourea (Y = S) derivative **3**.

The bisthiourea derivatives (Y = S) can be alkylated with a suitable alkylating agent, for example iodomethane, in an appropriate solvent such as ethanol, and at ambient temperature to reflux temperature to generate the dimethyl carbamimidothioates **5.**

As shown in SCHEME 1B, diamine **1** may also be reacted with imidates in a suitable solvent such as dioxane in the presence of acetic acid and sodium acetate at ambient temperature to generate the carboximidamides **7.**

As shown in SCHEME 1C, the diamine **1** is also amenable to acylation with various acyl halides in the presence of a suitable base such as triethylamine and in an appropriate solvent, for example dichloromethane, at ambient temperature to give the corresponding bisamide **9.**

The sulfonyl chlorides **11** shown in SCHEME 2, are known compounds. Reaction of **11** with a suitable amine in the presence of an appropriate base such as pyridine and in a solvent such as dichloromethane at ambient temperature affords the corresponding sulfonamide derivatives **13.**

The followings are the examples for the preparation, which is intended as an illustration of and not a limitation upon the scope of the invention.

### Example 1 Preparation of N,N"-(4-methyl-1,3-phenylene)bis[N'-(4-chlorophenyl)thiourea].

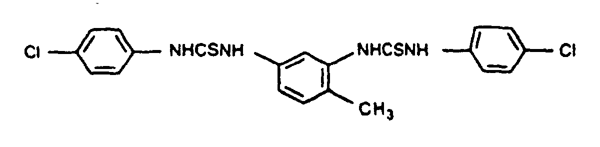

A solution of 4-chlorophenylisothiocyanate (1.39 g, 8.20 mmol) in dry dioxane (20 mL) is added dropwise, under nitrogen atmosphere, to a solution of 2,4-diaminotoluene (0.5 g, 4.10 mmol) in dry dioxane (20 mL). After 72 hours of stirring at room temperature the mixture is heated to 80 °C for 6 hours, and the solvent is eliminated under reduced pressure. The oily residue obtained is stirred with IsprOH (25 mL) for 4 hours. The white solid that precipitates is filtered and washed with n-hexane (3 x 25 mL) and ethyl ether (3 x 25 mL). The title compound is obtained as white powder. mp: 93-95 °C.
¹H-NMR (DMSO-d₆, 200 MHz) δ: 2.29; 7.15-7.25; 7.34; 7.47; 9.55; 9.61; 9.67; 9.93. IR: 1580 cm⁻¹.
MS-DIP (70 eV) m/z: 291.00; 168.95; 127.00.
ANAL: (C₂₁H₁₈Cl₂N₄S₂) C: Calcd, 54.66; Found, 54.41. H: Calcd, 3.90; Found, 3.92. N: Calcd, 12.14; Found, 11.85.

### Example 2 Preparation of N,N"-(4-methoxy-1,3-phenylene)bis[N'-(4-nitrophenyl)urea].

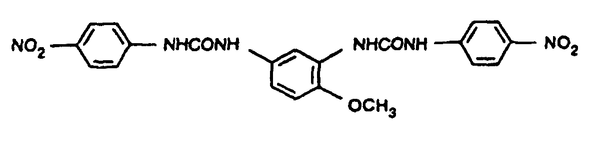

A solution of 4-nitrophenylisocyanate (1.39 g, 8.47 mmol) in dry dioxane (20 mL) is added dropwise, under N₂ atmosphere and with constant magnetic stirring, to a solution of 4-methoxy-m-phenylendiaminesulfate hydrate (1.00 g, 4.23 mmol) in dry dioxane (25 mL) and in the presence of triethylamine (1.2 mL). After 100 hours of stirring at room temperature, a precipitate appears. This precipitate is filtered and washed with H₂O (5 x 25 mL). The resulting solid is suspended in IsprOH (30 mL) and stirred at room temperature for 7 hours. It is filtered and washed successively with n-hexane (3 x 50 mL) and ethyl ether (3 x 50 mL). It is then dried. The title compound is obtained as yellow hygroscopic powder.
mp: 180-82 °C.
¹H-NMR (DMSO- d₆, 200 MHz) δ: 3.86; 6.99-7.19; 7.69; 8.23; 8.46; 8.87; 9.27; 10.05. IR: 1686 cm⁻¹.
ANAL: (C₂₁,H₁₈N₆O₇. 3/4 H₂O) C: Calcd, 52.55; Found, 52.34 H: Calcd, 4.07 Found, 4.21. N: Calcd, 17.52; Found, 17.32.

### Example 3 Preparation of N,N"-(4-methoxy-1,3-phenylene)bis[N'-(4-chlorophenyl)urea].

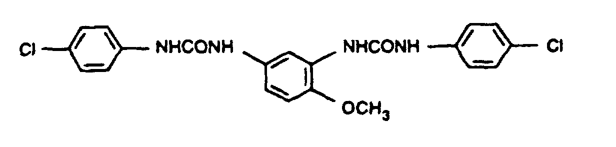

A solution of 4-chlorophenylisocyanate (1.30 g, 8.20 mmol) in dry dioxane (20 mL) is added dropwise, under N₂ atmosphere, to a solution of 4-methoxy-m-phenylenediaminesulfate hydrate (1.00 g, 4.23 mmol) in dry dioxane (20 mL) and in the presence of triethylamine (1.2 mL). After 72 hours of stirring at room temperature, a white precipitate appears. This precipitate is filtered and washed with H₂O (5 x 25 mL). The resulting solid is suspended in ethanol (30 mL) and stirred at room temperature for 3 hours. It is then filtered and recrystallized. The title compound is obtained as white powder. mp: >300 °C.
¹H-NMR (DMSO-d₆, 200 MHz) δ: 3.85; 6.94; 7.20; 7.30-7.35; 7.50; 8.20; 8.27; 8.62; 9.49.
IR: 3297; 1638 cm⁻¹.
ANAL: (C₂₁H₁₈Cl₂N₄O₃) C: Calcd, 56.63; Found, 56.54. H: Calcd, 4.04, Found, 4.06. N: Calcd, 12.58; Found, 12.41.

### Example 4 Preparation of N,N"-(4-methoxy-1,3-phenylene)bis[N'-(p-tolyl)urea).

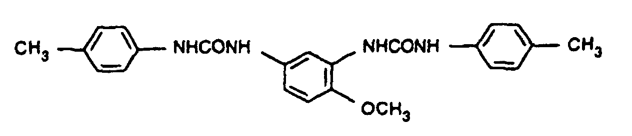

A solution of p-tolylisocyanate (1.12 g, 8.42 mmol) in dry dioxane (20 mL) is added dropwise, under N₂ atmosphere and with constant stirring, to a solution of 4-methoxy-m-phenylendiaminesulfate hydrate (1.00 g, 4.23 mmol) in dry dioxane (25 mL) and in the presence of triethylamine (1.2 mL). After 150 hours of stirring at room temperature, a precipitate appears. This precipitate is filtered and washed with H₂O (5 x 25 mL). The resulting solid is suspended in EtOH (30 mL) and stirred at room temperature for 4 hours. It is then filtered, washed successively with n-hexane (3 x 50 mL) and ethyl ether (3 x 50 mL), and dried. The title compound is obtained as white hygroscopic powder. mp: 162-64 °C.
¹H-NMR (DMSO-d₆, 200 MHz) δ 2.24; 3.84; 6.92; 7.05-7.93; 7.22; 7.33; 8.19; 8.36; 8.51; 9.24.
IR: 3296; 1640 cm⁻¹.
ANAL: (C₂₃H₂₄N₄O₃) C: Calcd, 68.32; Found, 67.96. H: Calcd, 5.94; Found 5.62. N: Calcd, 13.86; Found, 14.19.

### Example 5 Preparation of N,N"-(4-methoxy-1,3-phenylene)bis[N'-(4-chlorophenyl)thiourea].

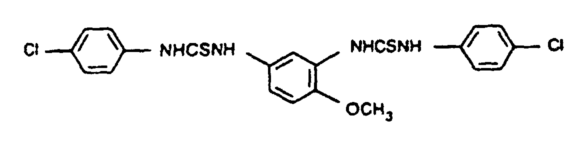

A solution of 4-chlorophenylisothiocyanate (1.43 g, 8.43 mmol) in dry dioxane (20 mL) is added dropwise, under N₂ atmosphere and with constant stirring, to a solution of 4-methoxy-m-phenylendiaminesulfate hydrate (1.00 g, 4.23 mmol) in dry dioxane (25 mL) and in the presence of triethylamine (1.2 mL). After 100 hours of stirring at room temperature, a precipitate appears. This precipitate is filtered and washed with H₂O (5 x 30 mL). The resulting solid is suspended in ethanol (30 mL) and stirred at room temperature for 5 hours. It is then filtered, washed successively with n-hexane (4 x 20 mL) and ethyl ether (4 x 20 mL) and dried. The title compound is obtained as white powder. mp: 114-16 °C.
¹H-NMR (DMSO-d₆, 200 MHz) δ: 3.84; 7.03; 7.23-7.36; 7.52; 8.02; 9.27.
IR: 1594 cm⁻¹.
ANAL: (C₂₁H₁₈Cl₂N₄O₂S₂.H₂O) C: Calcd, 50.91; Found, 50.85. H: Calcd. 4.04; Found, 4.03. N Calcd, 11.31; Found, 11.03.

### Example 6 Preparation of N,N"-(4-methoxy-1,3-phenylene)bis[N'-4-nitrophenyl)thiourea].

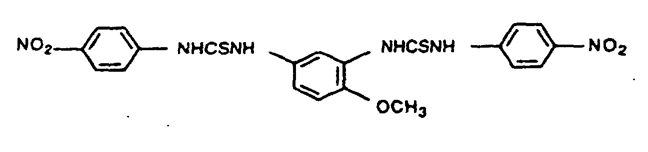

A solution of 4-nitrophenylisothiocyanate (1.52 g, 8.43 mmol) in dry dioxane (20 mL) is added dropwise, under N₂ atmosphere and with constant stirring, to a solution of 4-methoxy-m-phenylendiaminesulfate hydrate (1.00 g, 4.23 mmol) in dry dioxane (20 mL) and in the presence of triethylamine (1.2 mL). After 120 hours of stirring at room temperature, a yellow precipitate appears. This precipitate is filtered and washed with H₂O (7 x 25 mL). The resulting solid is suspended in IsprOH (25 mL) and stirred at room temperature for 5 hours. It is then filtered and recrystallized. The title compound is obtained as yellow powder. mp: 190-92 °C.
¹H-NMR (DMSO-d₆, 200 MHz) δ: 3.86; 7.09; 7.34; 7.81-8.01; 8.17; 9.68; 10.24-10.53. IR: 3337; 1606 cm⁻¹.
ANAL: (C₂₁H₁₈N₆O₅S₂) C: Calcd, 50.60; Found. 50.77. H: Calcd. 3.61; Found, 3.78. N: Calcd, 16.87; Found, 16.57.

### Example 7 Preparation of N,N"-(1,3-phenylene)bis[N-(p-tolyl)aminosulfonyl].

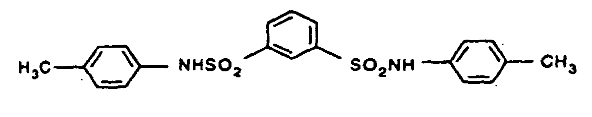

A solution of 1,3-benzendisulfonyl chloride (1 g, 363 mmol) in dry CH₂Cl₂ (20 mL) is added dropwise, under N₂ atmosphere, to a mixture of p-toluidine (1.55 g, 14.52 mmol) and pyridine (0.59 mL, 7.26 mmol), dissolved in dry CH₂Cl₂ (20 mL) and with magnetic stirring at room temperature. Stirring is maintained at room temperature for 17 days. When this time has elapsed, the solution is concentrated by rotatory evaporation and H₂O (50 mL) is added. The precipitate that forms is then isolated by vacuum filtration. This precipitate is then added to a solution of KOH 5% (50 mL). The mixture is stirred magnetically for 1 hour. Next, the mixture is filtered and HCI 35% is then added, until pH = 1. The precipitate that forms is collected by vacuum filtration. The title compound is obtained as white needles. mp: 189-90 °C.
¹H-NMR (DMSO-d₆, 200 MHz) δ: 2.19; 6.88; 7.00; 7.68; 7.86; 8.16; 10.32.
IR: 3246; 1510; 1323; 811; 681 cm⁻¹.
MS-DIP (70 eV) m/z: 416; 246; 106.
ANAL: (C₂₀H₂₀N₂O₄S₂) C: Calcd, 57.69; Found, 57.71. H: Calcd, 4.80; Found, 4.97. N: Calcd. 6.73: Found, 6.79.

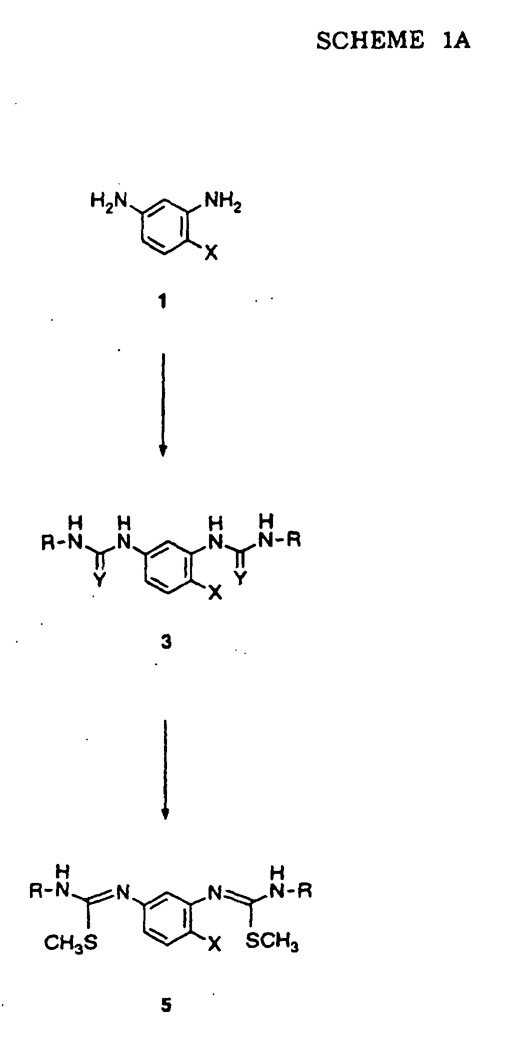

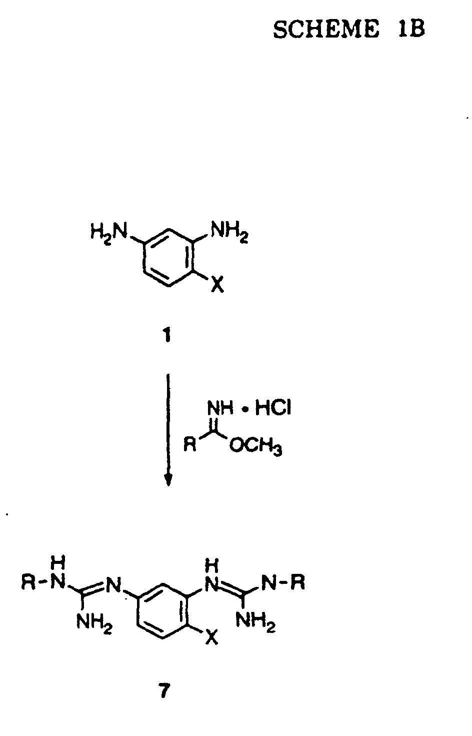

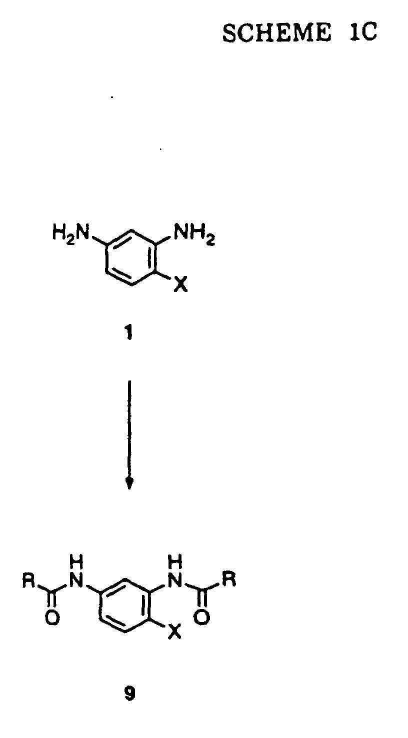

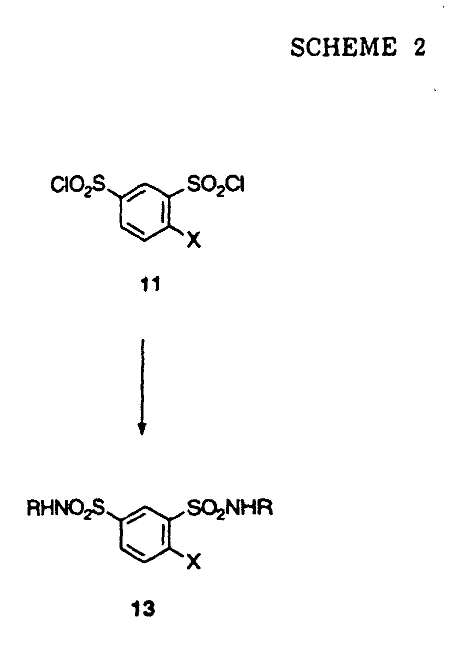

## Claims

1. A compound for treating herpes viral infections, wherein the compound is of formula II 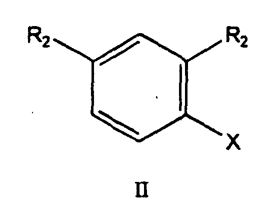 or a pharmaceutically acceptable salt thereof, wherein:
R₂ is
(a) 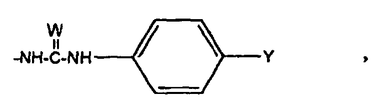 or
(b) 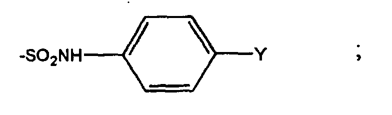
W is
(a) =O, or
(b) =S;
X is
(a) -H,
(b) -CH₃, or
(c) -OCH₃; and
Y is
(a) -CH₃,
(b) -Cl, or
(c) -NO₂.

2. A compound according to claim 1, which is
*N*,*N*"-(4-methoxy-1,3-phenylene)bis[*N*'-(4-nitrophenyl)urea],
*N*,*N*"-(4-methoxy-1,3-phenylene)bis[*N*'-(4-chlorophenyl)urea],
*N*,*N*"-(4-methoxy-1,3-phenylene)bis[*N*'-(*p*-tolyl)urea],
*N,N*"-(4-methyl-1,3-phenylene)bis[*N*'-(4-chlorophenyl)urea],
*N*,*N*"-(4-methoxy-1,3-phenylene)bis[*N*'-(4-chlorophenyl)thiourea],
*N*,*N*"-(4-methoxy-1,3-phenylene)bis[*N*'-(4-nitrophenyl)thiourea], or
*N*,*N*"-(1,-phenylene)bis[*N*'-(*p*-tolyl)aminosulfonyl].

3. A pharmaceutical composition for treating herpes viral infections, which comprises a compound according to claim 1 or claim 2, and a pharmaceutically acceptable carrier.

4. A composition according to claim 3, which comprises 0.05% to 25% by weight of the compound.

5. A composition according to claim 3, which comprises 0.1% to 10% by weight of the compound.

6. Use of a compound according to claim 1 or claim 2, for the manufacture of a topical medicament for treating herpes virus infections.

7. Use according to claim 6, wherein the herpes virus infection is herpes simplex type-1, herpes simplex type-2 or varicella zoster virus.

## Patentansprüche

1. Verbindung zur Behandlung von Herpesvirusinfektionen, wobei diese eine Verbindung der Formel II 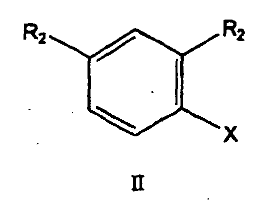 oder ein pharmazeutisch akzeptables Salz derselben ist, worin:
R₂
(a) 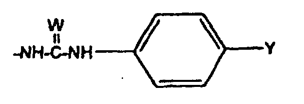 oder
(b) 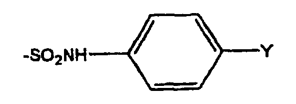 bedeutet;
W
(a) =O oder
(b) =S bedeutet;
X
(a) -H,
(b) -CH₃ oder
(c) -OCH₃ bedeutet; und
Y
(a) -CH₃,
(b) -Cl oder
(c) -NO₂ bedeutet.

2. Verbindung nach Anspruch 1, nämlich
N,N"-(4-Methoxy-1,3-phenylen)bis[N'-(4-nitrophenyl)harnstoff],
N,N"-(4-Methoxy-1,3-phenylen)bis[N'-(4-chlorphenyl)harnstoff],
N,N"-(4-Methoxy-1,3-phenylen)bis[N'-(p-tolyl)harnstoff],
N,N"-(4-Methyl-1,3-phenylen)bis[N'-(4-chlorphenyl)harnstoff],
N,N"-(4-Methoxy-1,3-phenylen)bis[N'-(4-chlorphenyl)thioharnstoff],
N,N"-(4-Methoxy-1,3-phenylen)bis[N'-(4-nitrophenyl)thioharnstoff] oder
N,N"-(1,3-Phenylen)bis[N'-(p-tolyl)aminosulfonyl].

3. Pharmazeutische Zusammensetzung zur Behandlung von Herpesvirusinfektionen, die eine Verbindung nach Anspruch 1 oder Anspruch 2 und einen pharmazeutisch akzeptablen Träger umfasst.

4. Zusammensetzung nach Anspruch 3, die 0,05 bis 25 Gew.-% der Verbindung umfasst.

5. Zusammensetzung nach Anspruch 3, die 0,1 bis 10 Gew.-% der Verbindung umfasst.

6. Verwendung einer Verbindung nach Anspruch 1 oder Anspruch 2 zur Herstellung eines topischen Medikaments zur Behandlung von Herpesvirusinfektionen.

7. Verwendung nach Anspruch 6, wobei die Herpesvirusinfektion das Herpex-simplex-Typ-1-, Herpex-simplex-Typ-2- oder Varicella-zoster-Virus ist.

## Revendications

1. Composé pour le traitement d'infections par des virus de l'herpès, ledit composé répondant à la formule II 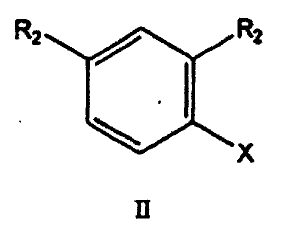 ou un de ses sels pharmaceutiquement acceptables, formule dans laquelle :
R₂ représente un groupe
(a) 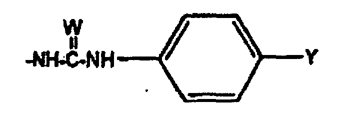 ou
(b) 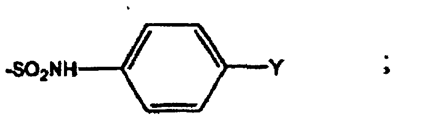
W représente
(a) =O, ou
(b) =S ;
X représente
(a) -H,
(b) -CH₃, ou
(c) -OCH₃ ; et
Y représente
(a) -CH₃,
(b) -Cl, ou
(c) -NO₂.

2. Composé suivant la revendication 1, qui est
la *N*,*N*"-(4-méthoxy-1,3-phénylène)bis[*N*'-(4-nitrophényl)urée],
la *N*,*N*"-(4-méthoxy-1,3-phénylène)bis[*N*'-(4-chlorophényl)urée],
la *N*,*N*"-(4-méthoxy-1,3-phénylène)bis[*N*'-(*p*-tolyl)urée],
la *N*,*N*"-(4-méthyl-1,3-phénylène)bis[*N*'-(4-chlorophényl)urée],
la *N*,*N*"-(4-méthoxy-1,3-phénylène)bis[*N*'-(4-chlorophényl)-thio-urée],
la *N, N"-* (4-méthoxy-1,3-phénylène)bis [*N*'-(4-nitrophényl)-thio-urée], ou
le *N*,*N*"-(1,3-phénylène)bis[*N*'-(*p*-tolyl)aminosulfonyle].

3. Composition pharmaceutique pour le traitement d'infections par des virus de l'herpès, qui comprend un composé selon la revendication 1 ou la revendication 2, et un support pharmaceutiquement acceptable.

4. Composition suivant la revendication 3, qui comprend 0,05 % à 25 % en poids du composé.

5. Composition suivant la revendication 3, qui comprend 0,1 % à 10 % du composé.

6. Utilisation d'un composé suivant la revendication 1 ou la revendication 2, pour la production d'un médicament topique destiné au traitement d'infections par des virus de l'herpès.

7. Utilisation suivant la revendication 6, dans laquelle l'infection par un virus de l'herpès est une infection par le virus Herpes simplex de type 1, le virus Herpes simplex de type 2 ou l'herpesvirus varicellae.
